# EUROPEAN PATENT SPECIFICATION

(11) **EP 2 735 262 B1**
(45) Date of publication and mention of the grant of the patent: **23.08.2017**
(21) Application number: 12848219.7
(22) Date of filing: 06.11.2012
(51) Int. Cl.: A61B 1/06, A61B 1/04, H04N 5/238

(54) **IMAGING DEVICE**
BILDGEBER
DISPOSITIF D'IMAGERIE

(30) Priority: 07.11.2011 JP 2011244035
(43) Date of publication of application: 28.05.2014
(73) Proprietor: Olympus Corporation, Tokyo 192-8507 (JP)
(72) Inventor: ONO, Wataru, Hachioji-shi, Tokyo 192-8507 (JP)
(74) Representative: Gunzelmann, Rainer
(86) International application number: PCT/JP2012/078746
(87) International publication number: WO 2013/069644

(56) References cited:
- JP-A- H11 155 808
- JP-A- 2004 321 605
- JP-A- 2006 191 236
- US-A- 5 812 187
- US-A1- 2008 027 278
- US-A1- 2010 069 713
- US-A1- 2010 245 619
- BRADLEY D ET AL: "Synchronization and rolling shutter compensation for consumer video camera arrays", COMPUTER VISION AND PATTERN RECOGNITION WORKSHOPS, 2009. CVPR WORKSHOPS 2009. IEEE COMPUTER SOCIETY CONFERENCE ON, IEEE, PISCATAWAY, NJ, USA, 20 June 2009 (2009-06-20), pages 1-8, XP031606989, ISBN: 978-1-4244-3994-2

## Description

### Field

The present invention relates to an imaging apparatus having an imaging element capable of outputting, as pixel information, electric signals that have been photoelectrically converted from pixels arbitrarily designated as reading targets from among a plurality of pixels for imaging.

### Background

Conventionally, in the medical field, an endoscopic system is used when organs of a subject such as a patient are observed. The endoscopic system has an insertion unit that has an elongated shape with flexibility and is inserted into a body cavity of the subject, an imaging unit that is provided at a distal end of the insertion unit and captures an in-vivo image, and a display unit that is able to display the in-vivo image captured by the imaging unit. When the in-vivo image is acquired using the endoscopic system, after the insertion unit is inserted into the body cavity of the subject, body tissue in the body cavity is illuminated with white light from the distal end of the insertion unit, and the imaging unit captures the in-vivo image. A user such a doctor observes the organs of the subject based on the in-vivo image displayed by the display unit.

### Citation List

### Patent Literature

Patent Literature 1: Japanese Laid-open Patent Publication No. 2009-192358

US 2010/245619 A1 concerns a fluoroscopy apparatus including an illumination portion irradiating an observation target with illumination light; a fluorescence image acquisition device that acquires fluorescence emitted from an observation target to acquire a fluorescence image; a fluorescence image-acquisition optical system that forms an image of the observation target using the fluorescence; a reference-light image acquisition device that acquires returning light from the observation region to acquire a reference image; a reference-light image-acquisition optical system that forms an image using the returning light; and an image correction section that corrects the fluorescence image by the reference image, wherein the product of an pixel density of the reference-light image acquisition device and the image-forming magnification of the reference-light image-acquisition optical system is less than the product of the pixel density of the fluorescence image acquisition device and the image-forming magnification of the fluorescence image-acquisition optical system.

US 2010/069713 A1 concerns an electronic endoscope system including an electronic endoscope having a CMOS image sensor on the tip of an insertion section, a light source device for illuminating the interior of a patient's body, and a processing device for reading out image signals from the CMOS image sensor. The electronic endoscope system can operate with a standard imaging mode and a special imaging mode. When the time taken to read out the image signals from all the pixels in the standard mode is defined as T, the light source device in the special imaging mode emits illumination light in every first half period T/2 while switching a wavelength of the illumination light between two different wavebands. In every second half period T/2, the processing device reads out the image signals from the half of the pixels.

### Summary

### Technical Problem

In the above-mentioned endoscopic system, illuminance of illumination light in an observation region varies depending on a distance from the distal end of the insertion unit. The illuminance decreases with the increasing distance from the distal end of the insertion unit, for example. Therefore, when the surface of the organs is observed from an oblique direction, a big difference in brightness arises between an upper portion of screen corresponding to a far point and a lower portion of the screen corresponding to a near point. Accordingly, the far point of the upper portion of the screen becomes darker.

Increase in intensity of illumination light to enlarge a dynamic range may solve such a problem. However, the increase in intensity of the illumination light may produce clipped white on a part of an image that is already bright before increasing the intensity of the illumination light. As a result, the dynamic range cannot be enlarged.

The present invention has been made in view of the foregoing and an object of the present invention is to provide an imaging apparatus that can enlarge a dynamic range of an image and obtain the image with proper brightness.

### Solution to Problem

To solve the problem described above and to achieve the object, an imaging apparatus of the invention includes the features of claim 1.

According to the imaging apparatus of the invention, in the above invention, the light source controller causes the light source unit to alternately emit the first illumination light and second illumination light, and the imaging controller causes the sensor unit to read the pixel information only during a time period in which one of the first illumination light and second illumination light is emitted.

In the above invention, the imaging apparatus of the invention further includes a light controller that performs light control of the first illumination light and the second illumination light. The light source controller causes the light source unit to alternately emit the first illumination light and the second illumination light in a cycle of the one-frame period, the imaging controller sets a first light control area where the first illumination light is controlled and a second light control area where the second illumination light is controlled, as different areas in one frame, and the light controller individually performs the light control in the first and second light control areas.

In the above invention, the imaging apparatus of the invention further includes an image synthesizing unit that generates a synthesized image by synthesizing, per pixel, the pixel information worth two screens chronologically read by the sensor unit. The light source controller causes the light source unit to alternately emit the first illumination light and the second illumination light in a cycle of the one-frame period.

According to the imaging apparatus of the invention, in the above invention, the imaging controller causes the sensor unit to: subject a plurality of one-dimensional lines parallel to each other which constitute the plurality of pixels, to light exposure per line; and sequentially perform reading of the pixel information in the line for which the light exposure has been completed.

According to the imaging apparatus of the invention, in the above invention, the one-dimensional lines are horizontal lines of the frame, and the imaging controller causes the sensor unit to sequentially perform light exposure and reading of the horizontal lines from an upper portion to a lower portion of the frame or from the lower portion to the upper portion.

According to the imaging apparatus of the invention, in the above invention, the one-dimensional lines are horizontal lines of the frame, and the imaging controller causes the sensor unit to sequentially perform light exposure and reading of the horizontal lines from a middle portion to both of an upper end portion and a lower end portion of the frame.

### Advantageous Effects of Invention

The present invention includes: a light source unit capable of emitting first illumination light and second illumination light that have illumination intensities different from each other as illumination light that illuminates a subject; a sensor unit in which a plurality of pixels each generating an electric signal by receiving light and performing photoelectric conversion are arranged on a two-dimensional surface, and that reads, as pixel information, the electric signals generated by pixels arbitrarily set as a reading target from among the plurality of pixels; an imaging controller that controls the sensor unit to read the pixel information; and a light source controller that controls the light source unit to emit any one of the first illumination light and second illumination light per one-frame period that is a time period necessary for the sensor unit to read the pixel information worth one screen. With this structure, it is possible to adjust the illumination light so as to be in a proper situation. Therefore, it is possible to enlarge a dynamic range of an image and obtain the image with proper brightness.

### Brief Description of Drawings

FIG. 1 is a diagram illustrating a schematic configuration of an endoscopic system having an imaging apparatus according to a first embodiment of the present invention.
FIG. 2 is a cross-sectional view illustrating a schematic internal configuration of a distal end portion of an endoscope included in the endoscopic system according to the first embodiment of the present invention.
FIG. 3 is a block diagram illustrating a functional configuration of main units of the endoscopic system according to the first embodiment of the present invention.
FIG. 4 is a diagram schematically illustrating an outline of an image acquiring method executable by the endoscopic system according to the first embodiment of the present invention, and tendencies of brightness of images acquired by the image acquiring method.
FIG. 5 is a diagram schematically illustrating an outline of an image acquiring method characteristic of the endoscopic system according to the first embodiment of the present invention, and tendencies of brightness of images acquired by to the image acquiring method.
FIG. 6 is a diagram illustrating an example of a case in which image reading in a pattern illustrated in FIG. 5 is effective.
FIG. 7 is a diagram illustrating a display example of an image acquired in a situation illustrated in FIG. 6 by the endoscopic system according to the first embodiment of the present invention.
FIG. 8 is a diagram illustrating an outline of an image acquiring method characteristic of an endoscopic system according a second embodiment of the present invention.
FIG. 9 is a block diagram illustrating a functional configuration of main units of an endoscopic system according to a third embodiment of the present invention.
FIG. 10 is a diagram illustrating an outline of an image acquiring method characteristic of the endoscopic system according the third embodiment of the present invention.
FIG. 11 is a diagram schematically illustrating an outline of an image acquiring method characteristic of an endoscopic system according to a fourth embodiment of the present invention, and tendencies of brightness of images acquired by the image acquiring method.
FIG. 12 is a diagram schematically illustrating details of a reading sequence in a transfer period according to the fourth embodiment of the invention.

### Description of Embodiments

Hereinafter, modes for carrying out the present invention (hereinafter, referred to as "embodiments") will be described with reference to the accompanying drawings. The drawings referred to in the following description are schematic, and dimensions, scaling, and the like may differ when the same object is illustrated in different drawings.

### (First Embodiment)

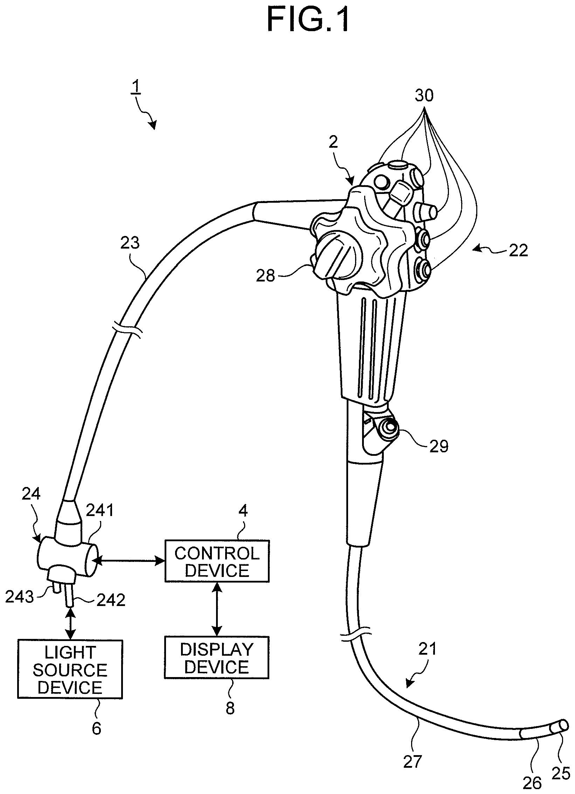

FIG. 1 is a diagram illustrating a schematic configuration of an endoscopic system having an imaging apparatus according to a first embodiment of the present invention. An endoscopic system 1 illustrated in FIG. 1 includes: an endoscope 2 that captures an in-vivo image of a subject by insertion of a distal end portion thereof into a body cavity of the subject; a control device 4 that has a function of performing a process on the in-vivo image acquired by the endoscope 2 and has a function of comprehensively controlling operations of the entire endoscopic system 1; a light source device 6 that is a light source unit generating illumination light emitted from a distal end of the endoscope 2, and a display device 8 that displays the in-vivo image subjected to image processing by the control device 4. The imaging apparatus according to the first embodiment is configured of the endoscope 2 and the control device 4.

The endoscope 2 includes: an insertion unit 21 that has an elongated shape with flexibility; an operating unit 22 that is connected at a proximal end side of the insertion unit 21 and receives input of operation signals; a universal cord 23 that extends in a direction different from a direction in which the insertion unit 21 extends from the operating unit 22 and has therein various cables connected to the control device 4 and the light source device 6; and a connector unit 24 that is provided at a distal end portion of the universal cord 23 and establishes connection of the endoscope 2 to the control device 4 and the light source device 6.

The insertion unit 21 includes a distal end portion 25 that has therein an imaging element to be described later, a curved portion 26 that is configured of a plurality of curved pieces and is freely bendable, and a flexible tube portion 27 that is connected at a proximal end side of the curved portion 26, has flexibility, and is elongated.

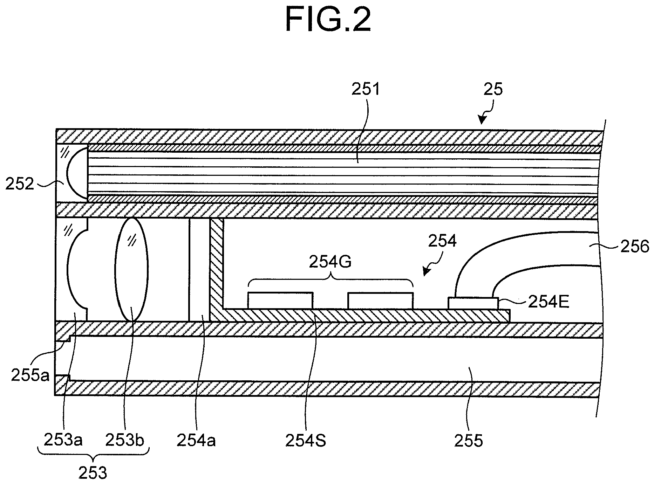

FIG. 2 is a cross-sectional view illustrating a schematic internal configuration of the distal end portion 25. As illustrated in FIG. 2, the distal end portion 25 includes: a light guide 251 that is configured using glass fiber or the like and forms an optical guide path of light generated by the light source device 6; an illumination lens 252 that is provided at a distal end of the light guide 251, an optical system 253 for condensing light; an imaging element 254 that is provided at an image formation position of the optical system 253, receives the light condensed by the optical system 253, photoelectrically converts the light into an electric signal, and performs a predetermined signal process on the electric signal; and a treatment tool channel 255 through which a treatment tool for the endoscope 2 is passed.

The optical system 253 is formed of two lenses 253a and 253b. The type or number of lenses forming the optical system 253 is not limited to those illustrated in FIG. 2.

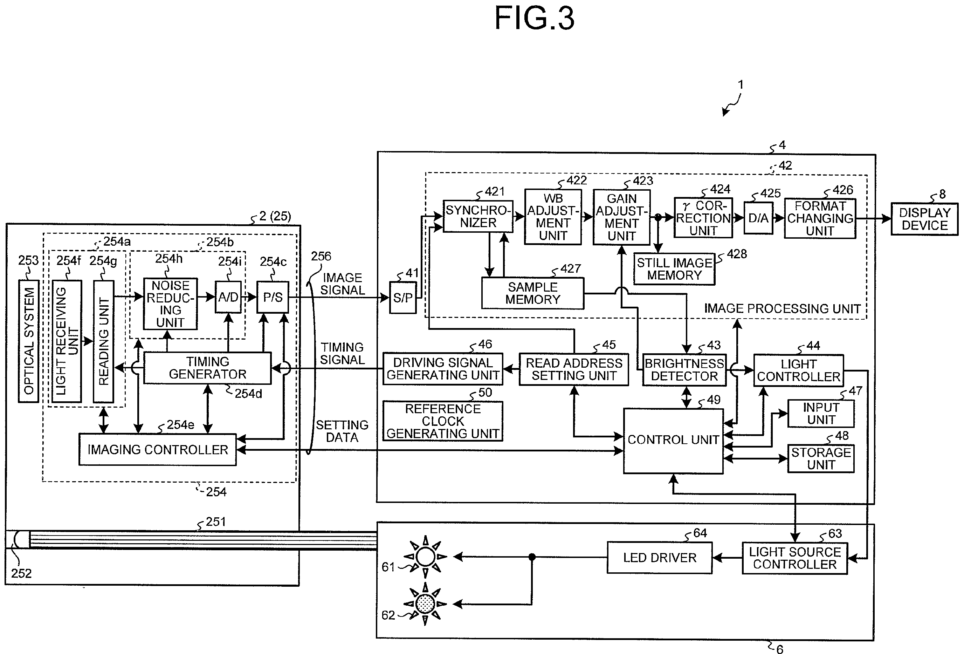

FIG. 3 is a block diagram illustrating a functional configuration of main units of the endoscopic system 1. A configuration of the imaging element 254 will be described with reference to FIG. 3. The imaging element 254 includes: a sensor unit 254a that performs the photoelectric conversion of the light from the optical system 253 and outputs the electric signal; an analog front end (AFE) unit 254b that performs noise removal and A/D conversion with respect to the electric signal output by the sensor unit 254a; a P/S converter 254c that performs parallel/serial conversion of a digital signal output by the analog front end unit 254b; a timing generator 254d that generates a driving timing pulse of the sensor unit 254a and pulses for various signal processing in the analog front end unit 254b and the P/S converter 254c; and an imaging controller 254e that controls operations of the imaging element 254. The imaging element 254 is a complementary metal oxide semiconductor (CMOS) image sensor.

The sensor unit 254a is connected to an IC circuit group 254G through a substrate 254S. The IC circuit group 254G includes a plurality of IC circuits having functions of the analog front end unit 254b, the P/S converter 254c, the timing generator 254d, and the imaging controller 254e.

The sensor unit 254a includes: a light receiving unit 254f in which a plurality of pixels, each having a photodiode accumulating an electric charge according to a light quantity and an amplifier amplifying the electric charge accumulated by the photodiode, are arranged in a two-dimensional matrix; and a reading unit 254g that reads, as pixel information, electric signals generated by pixels arbitrarily set as reading targets from among the plurality of pixels of the light receiving unit 254f. The light receiving unit 254f is provided with individual color filters for RGB for each pixel to enable acquirement of a color image.

The analog front end unit 254b includes a noise reducing unit 254h that reduces a noise component included in a signal, and an A/D converter 254i that performs A/D conversion on the noise-reduced signal. The noise reducing unit 254h reduces the noise, for example, using a correlated double sampling method. An auto gain control (AGC) circuit that automatically adjusts a gain of a signal to always keep a constant output level may be provided between the noise reducing unit 254h and the A/D converter 254i.

The imaging controller 254e controls various operations of the distal end portion 25 according to setting data received from the control device 4. The imaging controller 254e is configured using a central proccessing unit (CPU).

An electrode 254E provided on the substrate 254S is connected to a collective cable 256 bundled of a plurality of signal lines that transmit and receive electric signals to and from the control device 4. The plurality of signal lines include a signal line that transmits an image signal output by the imaging element 254 to the control device 4, and a signal line that transmits a control signal output by the control device 4 to the imaging element 254.

The operating unit 22 includes a curving knob 28 that curves the curved portion 26 in vertical and horizontal directions, a treatment tool insertion unit 29 through which a treatment tool such as a biopsy forceps or a laser probe is inserted into the body cavity, and a plurality of switches 30 for operating peripheral devices such as air supply means, water supply means, or gas supply means, in addition to the control device 4 and the light source device 6. The treatment tool inserted from the treatment tool insertion unit 29 comes out of an opening portion 255a through the treatment tool channel 255 of the distal end portion 25.

The universal cord 23 has therein at least the light guide 251 and the collective cable 256.

The connector unit 24 includes an electric contact point portion 241 that is connected to the control device 4, a light guide connector 242 that is freely-attachably and freely-detachably connected to the light source device 6, and an air supply sleeve 243 for sending air to a nozzle of the distal end portion 25.

Next, a configuration of the control device 4 will be described. The control device 4 includes an S/P converter 41, an image processing unit 42, a brightness detector 43, a light controller 44, a read address setting unit 45, a driving signal generating unit 46, an input unit 47, a storage unit 48, a control unit 49, and a reference clock generating unit 50.

The S/P converter 41 performs serial/parallel conversion on an image signal (digital signal) received from the distal end portion 25.

The image processing unit 42 generates an in-vivo image displayed by the display device 8 based on the parallel-mode image signal output from the S/P converter 41. The image processing unit 42 includes a synchronizer 421, a white balance (WB) adjustment unit 422, a gain adjustment unit 423, a γ correction unit 424, a D/A converter 425, a format changing unit 426, a sample memory 427, and a still image memory 428.

The synchronizer 421 inputs the image signal input as the pixel information in three memories (not illustrated) provided for each pixel, sequentially updates and holds a value in each memory correspondingly with an address of the pixel of the light receiving unit 254f read by the reading unit 254g, and synchronizes the image signals in the three memories as RGB image signals. The synchronizer 421 sequentially outputs the synchronized RGB image signals to the white balance adjustment unit 422, and outputs a part of the RGB image signals to the sample memory 427 for image analysis such as brightness detection.

The white balance adjustment unit 422 adjusts white balances of the RGB image signals.

The gain adjustment unit 423 adjusts gains of the RGB image signals. The gain adjustment unit 423 outputs the gain-adjusted RGB image signals to the γ correction unit 424, and outputs a part of the RGB signals to the still image memory 428 for still image display, enlarged image display, or weighted image display.

The γ correction unit 424 performs gradation correction (γ correction) on the RGB image signals correspondingly with the display device 8.

The D/A converter 425 converts the gradation-corrected RGB image signals output by the γ correction unit 424 into analog signals.

The format changing unit 426 changes the image signals converted into the analog signals, to a moving image file format, and outputs it to the display device 8. The AVI format, the MPEG format, or the like may be applied as the file format.

The brightness detector 43 detects a brightness level corresponding to each pixel, from the RGB image signals stored in the sample memory 427, records the detected brightness level in a memory provided therein, and outputs the detected brightness level to the control unit 49. In addition, the brightness detector 43 calculates a gain adjustment value and a light illumination amount based on the detected brightness level, outputs the gain adjustment value to the gain adjustment unit 423, and outputs the light illumination amount to the light controller 44.

The light controller 44, under the control of the control unit 49, sets a type, a light quantity, a light emission timing, an the like of light generated by the light source device 6 based on the light illumination amount calculated by the brightness detector 43 and transmits a light source synchronization signal including these set conditions to the light source device 6.

The read address setting unit 45 has a function of setting reading target pixels and a reading sequence in the light receiving unit of the sensor unit 254a. That is, the read address setting unit 45 has a function of setting addresses of pixels of the sensor unit 254a read by the analog front end unit 254b. In addition, the read address setting unit 45 outputs address information of the set reading target pixels to the synchronizer 421.

The driving signal generating unit 46 generates a driving timing signal for driving the imaging element 254, and transmits the timing signal to the timing generator 254d through a predetermined signal line included in the collective cable 256. The timing signal includes the address information of the reading target pixels.

The input unit 47 receives an input of various signals such as operation instruction signals for instructing operations of the endoscopic system 1.

The storage unit 48 is realized using a semiconductor memory such as flash memory or a dynamic random access memory (DRAM). The storage unit 48 stores various programs for operating the endoscopic system 1 and data including various parameters necessary for the operations of the endoscopic system 1.

The control unit 49 is configured using a central processing unit (CPU) or the like, and performs driving control of respective structural elements including the distal end portion 25 and the light source device 6, and input/output control of information with respect to the respective structural elements. The control unit 49 transmits setting data for imaging control to the imaging controller 254e through a predetermined signal line included in the collective cable 256. Herein, the setting data includes instruction information instructing an imaging speed (a frame rate) of the imaging element 254 and a reading speed of the pixel information from an arbitrary pixel of the sensor unit 254a, and transmission control information of the image information read by the analog front end unit 254b.

The reference clock generating unit 50 generates a reference clock signal that serves as a reference for operations of each structural element of the endoscopic system 1, and supplies the generated reference clock signal to each structural element of the endoscopic system 1.

Next, a configuration of the light source device 6 will be described. The light source device 6 includes a white light source 61, a special light source 62, a light source controller 63, and an LED driver 64.

The white light source 61 generates white illumination light generated by an LED or the like.

The special light source 62 generates, as special light, light of any of R, G, and B components that has been band-narrowed by a narrow band-pass filter and that has a wavelength band different from that of the white illumination light. As the special light generated by the special light source 62, there is narrow band imaging (NBI) illumination light of two kinds of bands, which are blue light and green light that have been band-narrowed so as to be easily absorbable by hemoglobin in blood.

The light source controller 63 controls an amount of electric current supplied to the white light source 61 or the special light source 62 according to the light source synchronization signal transmitted from the light controller 44.

The LED driver 64 supplies electric current to the white light source 61 or the special light source 62 under the control of the light source controller 63 to cause the white light source 61 or the special light source 62 to generate light.

The light generated by the white light source 61 or the special light source 62 is illuminated from a distal end of the distal end portion 25 to the outside through the light guide 251.

The display device 8 has a function of receiving the in-vivo image generated by the control device 4 from the control device 4 and displaying the in-vivo image. The display device 8 has a display such as a liquid crystal display or an organic EL display.

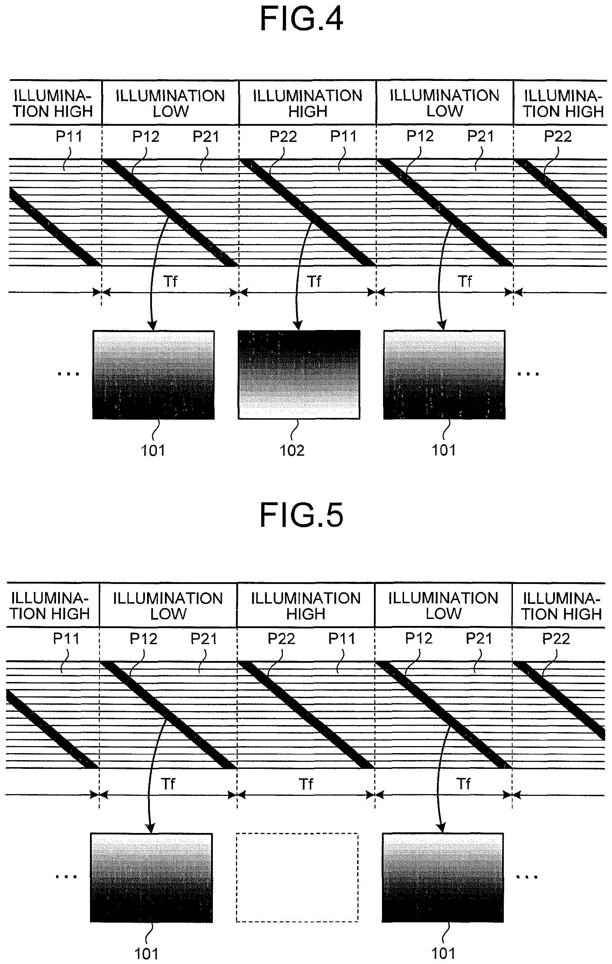

FIG. 4 is a diagram schematically illustrating an outline of an image acquiring method which is executable by the endoscopic system 1 having the configuration described above, and tendencies of brightness of images acquired by the image acquiring method. The imaging element 254 employs a focal-plane electronic shutter and thus if a plurality of frames are consecutively captured, reading of accumulated charges is sequentially performed one horizontal line by one horizontal line. Therefore, a time difference is generated between a horizontal line that is read first and a horizontal line that is read lastly. In the first embodiment, this time difference is considered as approximately equal to that worth one frame Tf.

In the example illustrated in FIG. 4, reading of pixels is performed starting from a horizontal line at a top portion of a screen, and sequentially to horizontal lines downward. In the first embodiment, the light source device 6 switches illumination intensity of illumination light of the same type (for example, white light) between a high intensity (High) and a low intensity (Low), in a cycle of one-frame period Tf. In this case, a light exposure time period worth one frame in the sensor unit 254a is a time period striding over a timing to switch the illumination intensity.

For example, in a light exposure period P11 illustrated in FIG. 4, a time period over which the illumination intensity is high is dominant in a horizontal line upper on the screen, but a time period over which the illumination intensity is low is dominant in a horizontal line lower on the screen. Accordingly, an image based on pixel information read by the reading unit 254g in a transfer period P12 of electric charges accumulated by light exposure of the sensor unit 254a in the light exposure period P11, tends to be brighter upper on the screen, and to gradually get darker downwards on the screen, as illustrated by an image 101 of FIG. 4.

In a light exposure period P21 illustrated in FIG. 4, a period over which the illumination intensity is low is dominant in a horizontal line upper on the screen, but a period over which the illumination intensity is high is dominant in a horizontal line lower on the screen. Accordingly, an image based on pixel information read by the reading unit 254g in a transfer period P22 of electric charges accumulated by light exposure of the sensor unit 254a in the light exposure period P21, tends to be dark upper on the screen, and to gradually get brighter downwards on the screen, as illustrated by an image 102 of FIG. 4.

The brightness and darkness of the screen explained herein describes a tendency of brightness of the screen due only to a difference in illuminance of the illumination light, and have no relation to brightness and darkness of an image of a subject when the image of that subject is captured. That is, a tendency of brightness when an actual subject is captured is not necessarily completely the same as the tendency of brightness and darkness illustrated by the image 101 or 102.

As described, when the illumination intensity is switched between high and low per image frame, influence of the illumination on the image is different for each frame. Thus, the imaging controller 254e causes the reading unit 254g to read only one of two kinds of images having brightness patterns different from each other so as to acquire the image every other frame.

FIG. 5 is a diagram schematically illustrating an outline of an image acquiring method characteristic of the endoscopic system 1 and the tendencies of brightness of the images acquired by the image acquiring method. For example, the reading unit 254g reads only the images 101 having the same brightness pattern of the screen under the control of the imaging controller 254e, and does not read the images (corresponding to the image 102 in FIG. 4) having a brightness pattern of the screen different from that of the images 101.

In the first embodiment, as illustrated in FIG. 5, since the sensor unit 254a sequentially acquires the images of the light exposure time periods having illumination intensity switching patterns that are the same as each other, it is possible to acquire images always having a constant pattern of image contrast.

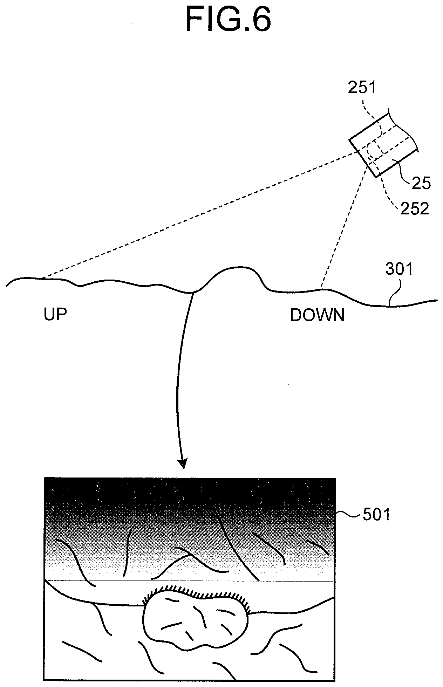

FIG. 6 is a diagram illustrating an example in which the image reading in the pattern illustrated in FIG. 5 is effective. In FIG. 6, the distal end portion 25 captures an image from diagonally above a subject 301. In this case, the closer to the distal end portion 25, the brighter an image 501 captured by temporally uniform illumination light gets; and the farther from the distal end portion 25, the darker the image 501 gets.

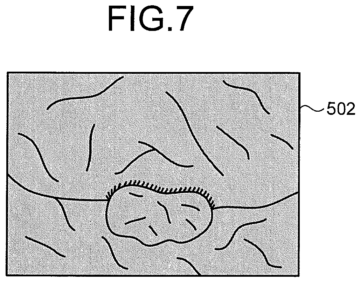

When an image with more uniform brightness than such an image 501 is to be captured, like the images 101 in FIG. 5, images may be obtained for which the sensor unit 254a is subjected to light exposure in the light exposure periods P11 over which the upper portion of the screen is bright and the lower portion of the screen is dark. FIG. 7 is a diagram illustrating a display example of an image acquired by light exposure of the sensor unit 254a in the light exposure period P11 in the endoscopic system 1 under the situation illustrated in FIG. 6. An image 502 illustrated in FIG. 7 schematically illustrates an image having an approximately uniform brightness of the screen.

In the first embodiment, by performing the image acquisition every other image frame period, the difference in illuminance between short-distance view and long-distance view of the illumination is corrected. Accordingly, even when a surface of a subject is viewed diagonally using the endoscope 2, it is possible to enlarge a dynamic range. Specifically, for example, under the situation illustrated in FIG. 6, by setting the long-distance view of the screen upper portion at a high sensitivity, an image with a wide dynamic range and ideal brightness from the near point to the far point is obtainable.

Only the images 102 in the reading time periods opposite to the case illustrated in FIG. 4 may be obtained. Such an image acquiring method is suitable for capturing an image in which a near end of an imaging field of view is bright and a far end thereof is dark.

In the first embodiment, by changing a ratio of highness and lowness for each frame of illumination generated by the light source device 6, it is possible to adjust a sensitivity balance between a short-distance view and a long-distance view of the screen.

According to the first embodiment of the present invention described above, by performing the illumination in which the brightness is changed per frame using the imaging element performing a shutter operation of the focal-plane shutter type, it is possible to acquire an image with different exposure times between the screen upper portion and the screen lower portion. Accordingly, it is possible to slant the sensitivity between the upper portion and the lower portion of the screen and to obtain an image with a wide dynamic range and ideal brightness.

In addition, in the first embodiment, the reading unit 254g reads only frames to be displayed, every other period, but the reading unit 254g may read all of the frames and the control device 4 may select frames to be displayed therefrom. In addition, after the reading unit 254g reads all of the frames, the imaging controller 254e may extract frames to be transmitted to the control device 4 as display targets.

In addition, in the first embodiment, the reading unit 254g may perform the reading per horizontal line from the lower portion to the upper portion of the screen.

### (Second Embodiment)

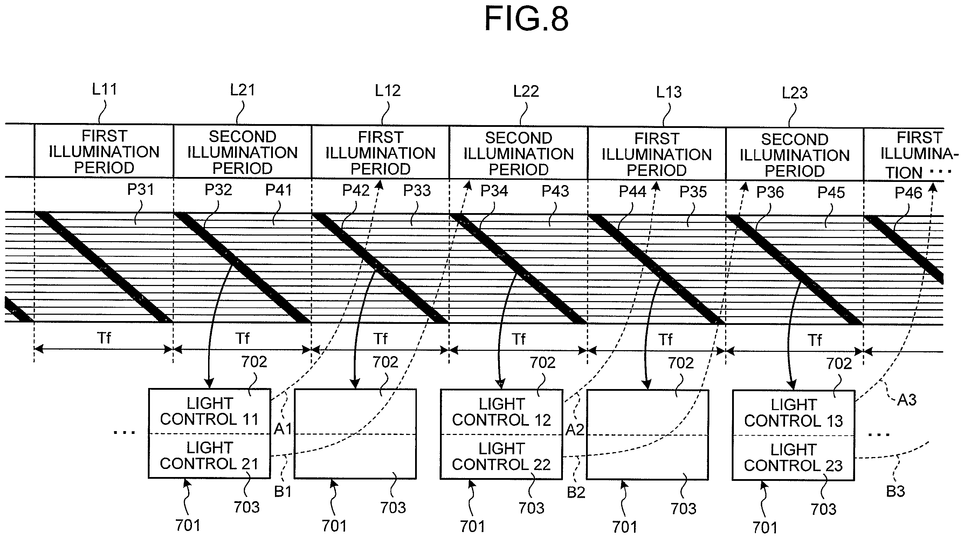

FIG. 8 is a diagram illustrating an outline of an image acquiring method characteristic of an endoscopic system according to a second embodiment of the present invention. A configuration of the endoscopic system according to the second embodiment is the same as the configuration of the endoscopic system 1 described above.

In the second embodiment, the light source device 6 alternately generates two different intensities in a constant cycle Tf as intensities of illumination light generated. Hereinafter, time periods over which the light source device 6 illuminates at two different intensities are referred to as a first illumination period and a second illumination period, respectively.

In the following description, an upper half area of an image 701 obtained by light exposure of the sensor unit 254a in a light exposure period (P31) striding over a time point changed from the first illumination period (for example, L11, and hereinafter, in this paragraph, what are in the parentheses may be associated among each other) to the second illumination period (L21) and by transmission by the sensor unit 254a of amounts of electric charge corresponding to the light exposure in a subsequent transmission time period (P32) is a first light control area 702, and a lower half area of the image 701 is a second light control area 703. The light controller 44 performs light control on the first light control area 702 as a light control target area of the first illumination period, and performs light control on the second light control area 703 as a light control target area of the second illumination period.

Results of the light control by the light controller 44 are transmitted to the light source controller 63. The light source controller 63 that has received the results of the light control of the light controller 44 reflects the results of the light control in the illumination intensities generated in the next first and second illumination periods.

The image acquiring method illustrated in FIG. 8 will be described more specifically. In FIG. 8, the light controller 44 performs light control of the first light control area 702 based on an amount of light exposure of the light exposure period P31 corresponding to the first illumination period L11 (light control 11). The light source controller 63 reflects results of the light control in the illumination intensity of the next first illumination period L12 (see a broken line A1).

The light controller 44 performs light control of the second light control area 703 based on the amount of light exposure of the light exposure period P31 corresponding to the second illumination period L21 (light control 21). The light source controller 63 reflects the result of the light control in the illumination intensity of the next second illumination period L22 (see a broken line B1).

Thereafter, the light controller 44 performs light control of the first light control area 702 based on an amount of light exposure (corresponding to amounts of electric charge transferred by the sensor unit 254a in a transfer period P34) of a light exposure period P33 corresponding to the first illumination period L12 subsequent to the second illumination period L21, without using an amount of exposure (corresponding to amounts of electric charge transferred by the sensor unit 254a in a transfer period P42) of a light exposure period P41 corresponding to the second illumination period L21 subsequent to the first illumination period L11 (light control 12). The light source controller 63 reflects the result of the light control in the illumination intensity of the next first illumination period L13 (see a broken line A2).

The light controller 44 performs light control of the second light control area 703 based on an amount of light exposure of the light exposure period P33 corresponding to the second illumination period L22 (light control 22). The light source controller 63 reflects a result of the light control in the illumination intensity of the next second illumination period L23 (see a broken line B2).

Thereafter, as described above, the light controller 44 performs the light control of the first illumination period in the first light control area 702, and performs the light control of the second illumination period in the second light control area 703.

According to the second embodiment of the present invention described above, similarly to the first embodiment described above, it is possible to obtain an image with a wide dynamic range and ideal brightness.

In addition, according to the second embodiment, since different light control areas are set for each light illumination period to perform light control independently, it becomes possible to set the brightness more finely.

In the second embodiment, if the first illumination period and the second illumination period are set to be of the same brightness, it is needless to say that it will be equal to a normal illumination method.

In addition, in the second embodiment, in the display device 8, images weighted for each of areas of different illumination periods may be synthesized and displayed, besides displaying an image of each read frame as-is.

### (Third Embodiment)

A third embodiment of the present invention is characterized in that two different illumination periods are alternately changed in a cycle similarly to the second embodiment, and an image synthesized of two adjacent frames is generated.

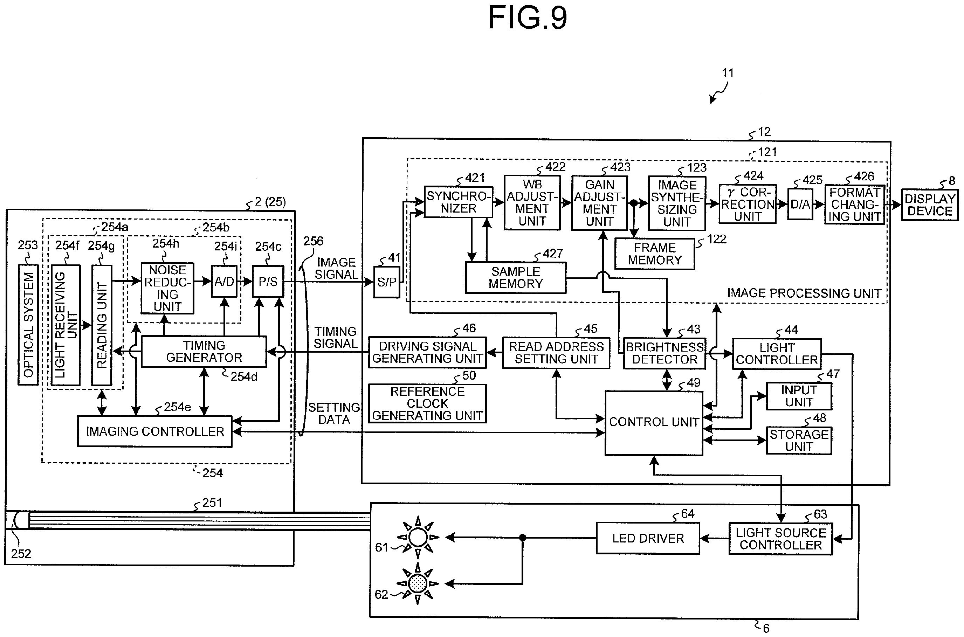

FIG. 9 is a block diagram illustrating a functional configuration of main elements of an endoscopic system according to the third embodiment of the present invention. In the endoscopic system 11 illustrated in FIG. 9, a configuration of an image processing unit included in a control device is different from that of the endoscopic system 1 described in the first embodiment.

An image processing unit 121 included in the control device 12 of the endoscopic system 11 has a frame memory 122 and an image synthesizing unit 123, in addition to the synchronizer 421, the white balance adjustment unit 422, the gain adjustment unit 423, the γ correction unit 424, the D/A converter 425, the format changing unit 426, and the sample memory 427.

The frame memory 122 temporarily stores an image subjected to gain adjustment by the gain adjustment unit 423.

The image synthesizing unit 123 adds the latest image subjected to gain adjustment by the gain adjustment unit 423 and an image of the previous frame for each pixel to generate a synthesized image.

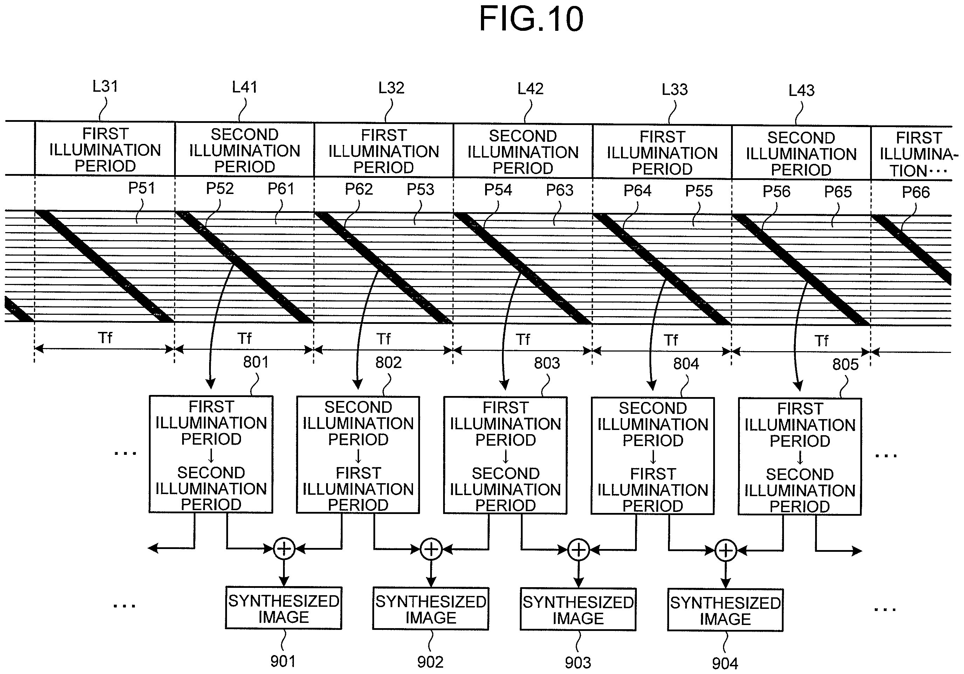

FIG. 10 is a diagram illustrating an outline of an image acquiring method characteristic of the endoscopic system 11. Similarly to the second embodiment, the light source device 6 alternately generates two different intensities in a constant cycle Tf. Hereinafter, periods over which the light source device 6 performs illumination with two different intensities are referred to as a first illumination period and a second illumination period, respectively.

In FIG. 10, the image synthesizing unit 123 generates a synthesized image 901, using: an image 801 obtained by light exposure of the sensor unit 254a in a light exposure period P51 striding over a time point at which a first illumination period L31 is switched to a second illumination period L41 and by transfer of an electric charge corresponding to the light exposure by the sensor unit 254a in the subsequent transfer period P52; and an image 802 obtained by light exposure of the sensor unit 254a in a light exposure period P61 striding over a time point at which the second illumination period L41 is switched to a first illumination period L32 and by transfer of an electric charge corresponding to the light exposure by the sensor unit 254a in the subsequent transfer period P62. This synthesized image 901 is generated by adding pixel values of corresponding pixels between the image 801 and the image 802. In addition, the image synthesizing unit 123 reads the first-acquired image 801 from among the two images 801 and 802 from the frame memory 122.

Subsequently, the image synthesizing unit 123 generates a synthesized image 902 by synthesizing the image 802 stored in the frame memory 122, with an image 803 obtained by light exposure of the sensor unit 254a in a light exposure period P53 striding over a time point at which the first illumination period L32 is switched to a second illumination period L42 and by transfer by the sensor unit 254a in the subsequent transfer period P54, as described above.

Thereafter, the image synthesizing unit 123 generates a synthesized image 903 by synthesizing the image 803 stored in the frame memory 122, with an image 804 obtained by light exposure of the sensor unit 254a in a light exposure period P63 striding over a time point at which the second illumination period L42 is switched to a first illumination period L33 and by transfer by the sensor unit 254a in the subsequent transfer period P64, as described above.

Subsequently, the image synthesizing unit 123 generates a synthesized image 904 by synthesizing the image 804 stored in the frame memory 122, with an image 805 obtained by light exposure of the sensor unit 254a in a light exposure period P55 striding over a time point at which the first illumination period L33 is switched to a second illumination period L43 and by transfer by the sensor unit 254a in the subsequent transfer period P56, as described above.

Thereafter, the image synthesizing unit 123 repeatedly performs the same processes as the processes described above to sequentially generate synthesized images.

According to the third embodiment of the present invention described above, the image synthesizing unit generates the synthesized image synthesized of two adjacent frame images, to thereby synthesize two illuminations with different brightnesses, and thus it is possible to enlarge the dynamic range with respect to the entire area of the image.

In the third embodiment, the light controller 44 may perform the light control based on the brightness in the synthesized frame, or may perform the light control with the same method as that of the second embodiment.

### (Fourth Embodiment)

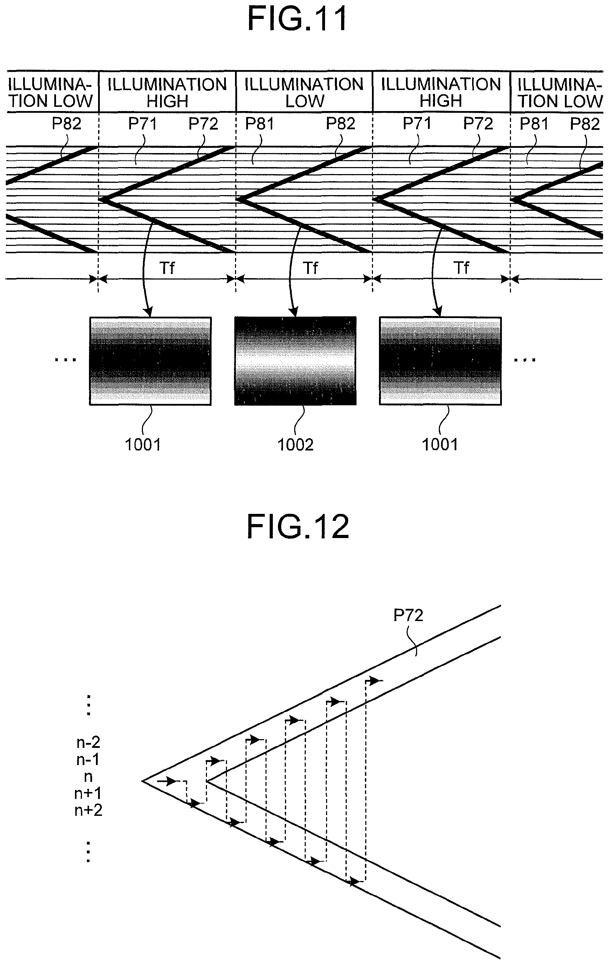

FIG. 11 is a diagram schematically illustrating an outline of an image acquiring method characteristic of an endoscopic system according to a fourth embodiment of the present invention and tendencies of brightness of images acquired according to the image acquiring method. A configuration of the endoscopic system according to the fourth embodiment is the same as that of the endoscopic system 1 described above.

In the fourth embodiment, an image reading sequence in a transfer period is different from those of the first and second embodiments. That is, in the fourth embodiment, the imaging element 254 starts reading from a horizontal line positioned in the middle of a screen, and thereafter alternately changes a horizontal line to be read toward both ends of the screen in its top-bottom direction.

FIG. 12 is a diagram schematically illustrating details of a reading sequence in a transfer period. In a transfer period P72 illustrated in FIG. 12, a horizontal line to be read is changed alternately down and up in a sequence of n-th horizontal line, (n+1)-th horizontal line, (n-1)-th horizontal line, (n+2)-th horizontal line, (n-2)-th horizontal line, and so on. Here, the natural number "n" has a value approximately half the number of horizontal lines the sensor unit 254a has.

In the fourth embodiment, the light source device 6 switches the generated illumination light between high and low at constant intensities, similarly to the first embodiment. Therefore, an image 1001, obtained by light exposure in a light exposure period P71 in which the illumination intensity is switched from low to high and by reading in a transfer period P72, is an image of which an upper end and a lower end of the screen are relatively bright, and it gradually gets darker toward the middle portion of the screen in the up-down direction. In an image 1002 for which light exposure is performed in a light exposure period P81 in which the illumination intensity is switched from high to low and which is read in a transfer period P82, the middle portion of the screen in the up-down direction is relative bright, and the image 1002 gradually gets darker toward the upper end portion and the lower end portion of the screen.

In the fourth embodiment, only either of the images 1001 and 1002 is read to generate a display image. For example, if an imaging field of view is bright in the middle portion of the screen in the up-down direction, the reading unit 254g may read only the images 1001. In addition, if an imaging field of view is dark in the middle portion of the screen in the up-down direction, the reading unit 254g may apply only the images 1002. Accordingly, similarly to the first embodiment described above, it is possible to generate an image having a uniform brightness.

According to the fourth embodiment of the present invention described above, similarly to the first embodiment described above, it is possible to obtain an image having a wide dynamic range and an ideal brightness.

In addition, according to the fourth embodiment, by performing the reading of the image alternatively from the middle frame of the image to both end portions in the up-down direction, it is possible to acquire an image added with a sensitivity slope in the up-down direction of the image.

The modes for embodying the invention have been described above, but the present invention is not limited only by the first to fourth embodiments described above. For example, in the present invention, the sequence in which the reading unit of the sensor unit reads the pixel information is not limited to the above description.

In addition, the present invention is applicable to laparoscopes used in endoscopy surgeries or the like. Since the inside of organs is observed from various directions in the endoscopy surgeries, brightness of a far point and brightness of a near point on a screen may drastically differ from each other similarly to the embodiments described above. Accordingly, it becomes possible to adjust the brightness of the imaging field of view as appropriate by applying the invention.

As described above, the present invention may include various embodiments which are not described herein, and various design modifications and the like within the scope of the technical concept described in the claims are possible.

### Reference Signs List

- 1, 11: endoscopic system
- 2: endoscope
- 4, 12: control device
- 6: light source device
- 8: display device
- 21: insertion unit
- 22: operating unit
- 25: distal end portion
- 26: curved portion
- 27: flexible tube portion
- 30: switch
- 41: S/P converter
- 42,: 121 image processing unit
- 43: brightness detector
- 44: light controller
- 45: read address setting unit
- 46: driving signal generating unit
- 47: input unit
- 48: storage unit
- 49: control unit
- 50: reference clock generating unit
- 61: white light source
- 62: special light source
- 63: light source controller
- 64: LED driver
- 122: frame memory
- 123: image synthesizing unit
- 242: light guide connector
- 251: light guide
- 252: illumination lens
- 253: optical system
- 254: imaging element
- 254a: sensor unit
- 254b: analog front end unit
- 254c: P/S converter
- 254e: imaging controller
- 254f: light receiving unit
- 254g: reading unit
- 256: collective cable
- 301: subject
- 702: first light control area
- 703: second light control area
- P11, P21, P31, P41, P51, P61: light exposure period
- P12, P22, P32, P42, P52, P62: transfer period

## Claims

1. An imaging apparatus, comprising:
a light source unit (61) configured to emit first illumination light and second illumination light that have illumination intensities different from each other as illumination light that illuminates a subject;
a sensor unit (254a) having a plurality of pixels on a plurality of one-dimensional lines, each of the plurality of pixels generating an electric signal by performing light exposure and photoelectric conversion, the plurality of one-dimensional lines being arranged parallel to one another on a two-dimensional surface and including first and last lines, the sensor unit (254a) being configured to read pixel information by a focal-plane method from the plurality of pixels so as to read from the first line to the last line in a one-frame period (Tf);
a light source controller (63) configured to cause the light source unit (61) to emit the first illumination light in a first illumination period that is a period between an emission start time of the first illumination light and an emission end time of the first illumination light, and to emit the second illumination light in a second illumination period that is a period between an emission start time of the second illumination light and an emission end time of the second illumination light in such a way that the first illumination period and the second illumination period switch alternately and continuously, the one-frame period (Tf) being set in the first illumination period and the second illumination period; and
an imaging controller (254e) configured to cause the sensor unit (254a) to perform light exposure in a period striding over the first illumination period and the second illumination period, and to selectively acquire one of a first image signal based on the pixel information read by the sensor unit (254a) in the first illumination period, and a second image signal based on the pixel information read by the sensor unit (254a) in the second illumination period, as different areas in one frame.

2. The imaging apparatus according to claim 1, further comprising:
a light controller configured to perform light control of the first illumination light and the second illumination light, wherein
the light source controller (63) is configured to cause the light source unit (61) to alternately emit the first illumination light and the second illumination light in a cycle of the one-frame period (Tf),
the imaging controller (254e) is configured to set a first light control area where the first illumination light is controlled and a second light control area where the second illumination light is controlled, as different areas in one frame, and
the light controller is configured to individually perform the light control in the first and second light control areas.

3. The imaging apparatus according to claim 1, further comprising:
an image synthesizing unit configured to generate a synthesized image by synthesizing, per pixel, the pixel information worth two screens chronologically read by the sensor unit, wherein
the light source controller is configured to cause the light source unit (61) to alternately emit the first illumination light and the second illumination light in a cycle of the one-frame period.

4. The imaging apparatus according to claim 1, wherein
the one-dimensional lines are horizontal lines of the frame, and
the imaging controller (254e) is configured to cause the sensor unit (254a) to sequentially perform light exposure and reading of the horizontal lines from an upper portion to a lower portion of the frame or from the lower portion to the upper portion.

5. The imaging apparatus according to claim 1, wherein
the one-dimensional lines are horizontal lines of the frame, and
the imaging controller (254e) is configured to cause the sensor unit (254a) to sequentially perform light exposure and reading of the horizontal lines from a middle portion to both of an upper end portion and a lower end portion of the frame.

6. The imaging apparatus according to claim 1, wherein the first illumination light and the second illumination light emitted by the light source unit (61) have a same wavelength component.

## Patentansprüche

1. Bildgeber, umfassend:
eine Lichtquelleneinheit (61), die so konfiguriert ist, dass sie ein erstes Beleuchtungslicht und ein zweites Beleuchtungslicht, die in Bezug aufeinander unterschiedliche Beleuchtungsintensitäten aufweisen, als Beleuchtungslicht emittiert, das ein Subjekt beleuchtet;
eine Sensoreinheit (254a) mit einer Mehrzahl von Pixeln auf einer Mehrzahl von eindimensionalen Linien, wobei jede der Mehrzahl von Pixeln durch Durchführen einer Lichtexposition und photoelektrischen Umwandlung ein elektrisches Signal erzeugt, wobei die Mehrzahl von eindimensionalen Linien parallel zueinander auf einer zweidimensionalen Oberfläche angeordnet ist und erste und letzte Linien umfasst, wobei die Sensoreinheit (254a) so konfiguriert ist, dass sie Pixelinformationen durch ein Fokalebenenverfahren aus der Mehrzahl von Pixeln liest, um in einer Ein-Frame-Periode (Tf) von der ersten Linie zur letzten Linie zu lesen;
eine Lichtquellensteuereinheit (63), die so konfiguriert ist, dass sie die Lichtquelleneinheit (61) dazu veranlasst, das erste Beleuchtungslicht in einer ersten Beleuchtungsperiode zu emittieren, wobei es sich um eine Periode zwischen einem Emissionsbeginnzeitpunkt des ersten Beleuchtungslichts und einem Emissionsendzeitpunkt des ersten Beleuchtungslichts handelt, und das zweite Beleuchtungslicht in einer zweiten Beleuchtungsperiode zu emittieren, wobei es sich um eine Periode zwischen einem Emissionsbeginnzeitpunkt des zweiten Beleuchtungslichts und einem Emissionsendzeitpunkt des zweiten Beleuchtungslichts handelt, so dass die erste Beleuchtungsperiode und die zweite Beleuchtungsperiode abwechselnd und kontinuierlich wechseln, wobei die Ein-Frame-Periode (Tf) in der ersten Beleuchtungsperiode und der zweiten Beleuchtungsperiode eingestellt ist; und
eine Bildgebungssteuereinheit (254e), die so konfiguriert ist, dass sie die Sensoreinheit (254a) dazu veranlasst, eine Lichtexposition in einer Periode durchzuführen, die die erste Beleuchtungsperiode und die zweite Beleuchtungsperiode überschreitet, und eines aus einem ersten Bildsignal auf Basis der von der Sensoreinheit (254a) gelesenen Pixelinformationen in der ersten Beleuchtungsperiode und eines zweiten Bildsignals auf Basis der von der Sensoreinheit (254a) gelesenen Pixelinformationen in der zweiten Beleuchtungsperiode als unterschiedliche Bereiche in einem Frame selektiv erfasst.

2. Bildgeber nach Anspruch 1, ferner umfassend:
eine Lichtsteuereinheit, die so konfiguriert ist, dass sie eine Lichtsteuerung des ersten Beleuchtungslichts und des zweiten Beleuchtungslichts durchführt, wobei:
dass die Lichtquellensteuereinheit (63) so konfiguriert ist, dass sie die Lichtquelleneinheit (61) dazu veranlasst, das erste Beleuchtungslicht und das zweite Beleuchtungslicht in einem Zyklus der Ein-Frame-Periode (Tf) abwechselnd zu emittieren,
dass die Bildgebungssteuereinheit (254e) so konfiguriert ist, dass sie einen ersten Lichtsteuerbereich, in dem das erste Beleuchtungslicht gesteuert wird, und einen zweiten Lichtsteuerbereich, in dem das zweite Beleuchtungslicht gesteuert wird, als unterschiedliche Bereiche in einem Frame einstellt, und
dass die Lichtsteuereinheit so konfiguriert ist, dass sie die Lichtsteuerung im ersten und im zweiten Lichtsteuerbereich individuell durchführt.

3. Bildgeber nach Anspruch 1, ferner umfassend:
eine Bildsynthetisierungseinheit, die so konfiguriert ist, dass sie ein synthetisiertes Bild durch Synthetisieren der Pixelinformationen im Wert von zwei Bildschirmen, die von der Sensoreinheit chronologisch gelesen werden, pro Pixel erzeugt, wobei:
die Lichtquellensteuereinheit so konfiguriert ist, dass sie die Lichtquelleneinheit (61) dazu veranlasst, das erste Beleuchtungslicht und das zweite Beleuchtungslicht in einem Zyklus der Ein-Frame-Periode abwechselnd zu emittieren.

4. Bildgeber nach Anspruch 1, wobei:
die eindimensionalen Linien horizontale Linien des Frames sind und
die Bildgebungssteuereinheit (254e) so konfiguriert ist, dass sie die Sensoreinheit (254a) dazu veranlasst, eine Lichtexposition und ein Lesen der horizontalen Linien von einem oberen Abschnitt zu einem unteren Abschnitt des Frames oder vom unteren Abschnitt zum oberen Abschnitt sequentiell durchzuführen.

5. Bildgeber nach Anspruch 1, wobei:
die eindimensionalen Linien horizontale Linien des Frames sind und
die Bildgebungssteuereinheit (254e) so konfiguriert ist, dass sie die Sensoreinheit (254a) dazu veranlasst, eine Lichtexposition und ein Lesen der horizontalen Linien von einem Mittelabschnitt zu sowohl einem oberen Endabschnitt als auch einem unteren Endabschnitt des Frames sequentiell durchzuführen.

6. Bildgeber nach Anspruch 1, wobei das erste Beleuchtungslicht und das zweite Beleuchtungslicht, die von der Lichtquelleneinheit (61) ausgegeben werden, eine gleiche Wellenlängenkomponente aufweisen.

## Revendications

1. Appareil d'imagerie, comprenant :
une unité de source lumineuse (61) configurée pour émettre une première lumière d'éclairage et une seconde lumière d'éclairage qui présentent des intensités d'éclairage différentes l'une de l'autre en tant que lumière d'éclairage qui éclaire un sujet ;
une unité de capteur (254a) présentant une pluralité de pixels sur une pluralité de lignes unidimensionnelles, chacun de la pluralité de pixels générant un signal électrique par réalisation d'une exposition à la lumière et d'une conversion photoélectrique, la pluralité de lignes unidimensionnelles étant agencées parallèles les unes aux autres sur une surface bidimensionnelle et comprenant des première et dernière lignes, l'unité de capteur (254a) étant configurée pour lire des informations de pixel par un procédé à plan focal à partir de la pluralité de pixels de manière à lire de la première ligne à la dernière ligne dans une période d'une trame (Tf) ;
un contrôleur de source lumineuse (63) configuré pour amener l'unité de source lumineuse (61) à émettre la première lumière d'éclairage dans une première période d'éclairage qui est une période entre un temps de début d'émission de la première lumière d'éclairage et un temps de fin d'émission de la première lumière d'éclairage, et à émettre la seconde lumière d'éclairage dans une seconde période d'éclairage qui est une période entre un temps de début d'émission de la seconde lumière d'éclairage et un temps de fin d'émission de la seconde lumière d'éclairage d'une façon telle que la première période d'éclairage et la seconde période d'éclairage commutent en alternance et en continu, la période d'une trame (Tf) étant réglée dans la première période d'éclairage et la seconde période d'éclairage ; et
un contrôleur d'imagerie (254e) configuré pour amener l'unité de capteur (254a) à réaliser une exposition à la lumière dans une période s'étalant sur la première période d'éclairage et la seconde période d'éclairage, et à acquérir de façon sélective l'un d'un premier signal d'image basé sur des informations de pixel lues par l'unité de capteur (254a) dans la première période d'éclairage, et d'un second signal d'image basé sur des informations de pixel lues par l'unité de capteur (254a) dans la seconde période d'éclairage, en tant que différentes zones dans une trame.

2. Appareil d'imagerie selon la revendication 1, comprenant en outre :
un contrôleur de lumière configuré pour réaliser une commande de lumière de la première lumière d'éclairage et de la seconde lumière d'éclairage, dans lequel :
le contrôleur de source lumineuse (63) est configuré pour amener l'unité de source lumineuse (61) à émettre en alternance la première lumière d'éclairage et la seconde lumière d'éclairage dans un cycle de la période d'une trame (Tf),
le contrôleur d'imagerie (254e) est configuré pour régler une première zone de commande de lumière où la première lumière d'éclairage est commandée et une seconde zone de commande de lumière où la seconde lumière d'éclairage est commandée, en tant que différentes zones dans une trame, et
le contrôleur de lumière est configuré pour réaliser de façon individuelle la commande de lumière dans les première et seconde zones de commande de lumière.

3. Appareil d'imagerie selon la revendication 1, comprenant en outre :
une unité de synthèse d'image configurée pour générer une image synthétisée par synthèse, par pixel, des informations de pixel valant deux écrans chronologiquement lus par l'unité de capteur, dans lequel :
le contrôleur de source lumineuse est configuré pour amener l'unité de source lumineuse (61) à émettre en alternance la première lumière d'éclairage et la seconde lumière d'éclairage dans un cycle de la période d'une trame.

4. Appareil d'imagerie selon la revendication 1, dans lequel :
les lignes unidimensionnelles sont des lignes horizontales de la trame, et
le contrôleur d'imagerie (254e) est configuré pour amener l'unité de capteur (254a) à réaliser de manière séquentielle l'exposition à la lumière et la lecture des lignes horizontales depuis une partie supérieure vers une partie inférieure de la trame ou depuis la partie inférieure vers la partie supérieure.

5. Appareil d'imagerie selon la revendication 1, dans lequel :
les lignes unidimensionnelles sont des lignes horizontales de la trame, et
le contrôleur d'imagerie (254e) est configuré pour amener l'unité de capteur (254a) à réaliser de manière séquentielle l'exposition à la lumière et la lecture des lignes horizontales depuis une partie intermédiaire vers à la fois une partie d'extrémité supérieure et une partie d'extrémité inférieure de la trame.

6. Appareil d'imagerie selon la revendication 1, dans lequel la première lumière d'éclairage et la seconde lumière d'éclairage émises par l'unité de source lumineuse (61) présentent une même composante de longueur d'onde.
